# EUROPEAN PATENT SPECIFICATION

(11) **EP 1 416 923 B1**
(45) Date of publication and mention of the grant of the patent: **05.08.2009**
(21) Application number: 02752714.2
(22) Date of filing: 07.08.2002
(51) Int. Cl.: A61K 8/42, A61K 8/69, A61Q 7/02, A61K 31/17, A61K 31/198

(54) **REDUCTION OF HAIR GROWTH**
VERMINDERUNG DES HAARWUCHSES
REDUCTION DE CROISSANCE CAPILLAIRE

(30) Priority: 10.08.2001 US 311600 P
(43) Date of publication of application: 12.05.2004
(73) Proprietor: The Gillette Company, Boston, Massachusetts 02199 (US)
(72) Inventor: STYCZYNSKI, Peter, Wrentham, MA 02093 (US); AHLUWALIA, Gurpreet, S., Newton, MA 20459 (US); SHANDER, Douglas, Acton, MA 01720 (US)
(74) Representative: Wilding, Richard Alan
(86) International application number: PCT/US2002/024963
(87) International publication number: WO 2003/013496

(56) References cited:
- WO-A-00/50002
- WO-A-01/54654
- WO-A-02/07697
- WO-A-92/10164
- WO-A-94/21216
- US-A- 4 720 489
- US-A- 5 132 293
- US-A- 5 648 394

## Description

### BACKGROUND

The invention relates to reducing hair growth in mammals, particularly for cosmetic purposes.

A main function of mammalian hair is to provide environmental protection. However, that function has largely been lost in humans, in whom hair is kept or removed from various parts of the body essentially for cosmetic reasons. For example, it is generally preferred to have hair on the scalp but not on the face.

Various procedures have been employed to remove unwanted hair, including shaving, electrolysis, depilatory creams or lotions, waxing, plucking, and therapeutic antiandrogens. These conventional procedures generally have drawbacks associated with them. Shaving, for instance, can cause nicks and cuts, and can leave a perception of an increase in the rate of hair regrowth. Shaving also can leave an undesirable stubble. Electrolysis, on the other hand, can keep a treated area free of hair for prolonged periods of time, but can be expensive, painful, and sometimes leaves scarring. Depilatory creams, though very effective, typically are not recommended for frequent use due to their high irritancy potential. Waxing and plucking can cause pain, discomfort, and poor removal of short hair. Finally, antiandrogens -- which have been used to treat female hirsutism -- can have unwanted side effects.

It has previously been disclosed that the rate and character of hair growth can be altered by applying to the skin inhibitors of certain enzymes. These inhibitors include inhibitors of 5-alpha reductase, ornithine decarboxylase, S-adenosylmethionine decarboxylase, gamma-glutamyl transpeptidase, and transglutaminase. See, for example, Breuer et al., U.S. Pat. 4,885,289; Shander, U.S. Pat. 4,720,489; Ahluwalia, U.S. Pat. 5,095,007; Ahluwalia et al., U.S. Pat. 5,096,911; and Shander et al., U.S. Pat. 5,132,293.

α-Difluoromethylomithine (DFMO) is an irreversible inhibitor of ornithine decarboxylase (ODC), a rate-limiting enzyme in the *de novo* biosynthesis of putrescine, spermidine, and spermine. The role of these polyamines in cellular proliferation is not yet well understood. However, they seem to play a role in the synthesis and/or regulation of DNA, RNA and proteins. High levels of ODC and polyamines are found in cancer and other cell types that have high proliferation rates.

DFMO binds the ODC active site as a substrate. The bound DFMO is then decarboxylated and converted to a reactive intermediate that forms a covalent bond with the enzyme, thus preventing the natural substrate ornithine from binding to the enzyme. Cellular inhibition of ODC by DFMO causes a marked reduction in putrescine and spermidine and a variable reduction in spermine, depending on the length of treatment and the cell type. Generally, in order for DFMO to cause significant antiproliferative effects, the inhibition of polyamine synthesis must be maintained by continuous inhibitory levels of DFMO because the half-life of ODC is about 30 min, one of the shortest of all known enzymes.

A skin preparation containing DFMO (sold under the name Vaniqa^{®} by Bristol Myers Squibb), has recently been approved by the Food and Drug Administration (FDA) for the treatment of unwanted facial hair growth in women. Its topical administration in a cream based vehicle has been shown to reduce the rate of facial hair growth in women. Vaniqa^{®} facial cream includes a racemic mixture of the "D-" and "L-" enantiomers of DFMO (i.e., D,L-DFMO) in the monohydrochloride form at a concentration of 13.9% by weight active (15%, as monohydrochloride monohydrate). The recommended treatment regimen for Vaniqa^{®} is twice daily. The cream base vehicle in Vaniqa^{®} is set out in Example 1 of U.S. 5,648,394. The cream base includes 2.5% ceteareth-20. Ceteareth-20 is a blend of two polyoxyethylene ethers of alkyl alcohols, having the chemical formulas CH₃(CH₂)₁₅(OCH₂CH₂)_{b} OH and CH₃ (CH₂)₁₇(OCH₂CH₂)_{b} OH, where b has an average value of 20.

It generally takers about eight weeks of continuous treatment before the hair growth-inhibiting efficacy of Vaniqa^{®} cream becomes apparent. Vaniqa^{®} cream has been shown to decrease hair growth an average of 47%. In one study, clinical successes were observed in 35% ofwomen treated with Vaniqa^{®} cream. These women exhibited marked improvement or complete clearance of their condition as judged by physicians scoring a decrease in visibility of facial hair and a decrease in skin darkening caused by hair. Another 35% of the women tested experienced some improvement in their condition. However, there were some women who exhibited little or no response to treatment.

Accordingly, although Vaniqa^{®} cream is an effective product, it would be even more effective if it provided an earlier onset of hair growth inhibition (i.e., exhibited efficacy earlier than eight weeks) and/or exhibited an increased clinical success rate (i.e., exhibited efficacy in a greater percentage of users). Such improved results cannot be obtained by simply increasing the concentration of D,L-DFMO in the cream vehicle. First, increasing the concentration of D,L-DFMO above about 14% can cause increased stinging of the skin and/or can leave a residue, making it aesthetically unacceptable. Second, it is difficult to formulate compositions with an active concentration above about 15% because significantly higher concentrations of D,L-DFMO are not adequately soluble in the vehicle or destabilize the emulsion.

Molecules that are identical to each other in chemical structural formula and yet are not superimposable upon each other are enantiomers. In terms of their physiochemical properties enantiomers differ only in their ability to rotate the plane of plane-polarized light, and this property is frequently used in their designation. Those entiomers that rotate plane-polarized light to the right are termed dextrorotatory, indicated by either a (+) - or d- or D-before the name of the compound; those that rotate light to the left are termed laevorotatory indicated by a (-)- or 1- or L- prefix. A racemic mixture is indicated by either a (±) - or d,1- or D,L- prefix. By another convention (or nomenclature), the R,S or the sequence rule can be used to differentiate enantiomers based on their absolute configuration. Using this system the L-DFMO corresponds to the R-DFMO, and the D-DFMO corresponds to the S-DFMO. Enantiomers are physiochemically similar in that they have similar melting points, boiling points, relative solubility, and chemical reactivity in an achiral environment. A racemate is a composite of equal molar quantities of two enantiomeric species, often referred to as the DL-form. Individual enantiomers of chiral molecules may possess different pharmacological profiles, i.e., differences in pharmacokinetics, toxicity, efficacy, etc.

### SUMMARY

The present invention provides a method (typically a cosmetic method) of reducing human hair growth by applying to the skin, in an amount effective to reduce hair growth, a dermatologically acceptable topical composition including α-difluoromethylornithine (DFMO) and a dermatologically acceptable vehicle. The vehicle includes urea. The vehicle may include, for example, from 0.1% to 20% urea by weight, preferably from 1% to 12% urea by weight, more preferably from 2% to 10% urea by weight, and most preferably 4% to 10% urea by weight. Without being bound by any theory, it is believed that the urea enhances the water holding capacity of the skin, which in turn leads to enhanced DFMO absorption. The unwanted hair growth may be undesirable from a cosmetic standpoint or may result, for example, from a disease or an abnormal condition (e.g., hirsutism).

The preferred vehicle also optionally includes a polyoxyethylene ether having the chemical formula R(OCH₂CH₂)_{b}OH, where R is a saturated or unsaturated alkyl group including from 6 to 22 carbon atoms and b has an average value of from 2 to 200. Preferably the alkyl group includes from 8 to 20, more preferably from 10 to 18, carbon atoms and b is from 2 to 100, more preferably from 2 to 50, most preferably from 2 to 30. Without being bound by any theory, it is believed that the polyoxyethylene ether disrupts, solubilizes, and/or emulsifies the lipid component of the skin, leading to enhanced skin absorption of the DFMO. Preferred vehicles include from 0.1% to 20%, more preferably from 1% to 12%, and most preferably from 4% or 5% to 12%, of the polyoxyethylene ether by weight.

For purposes ofthis application, the vehicle includes all components of the composition except the DFMO. DFMO, as used herein, includes DFMO itself and pharmaceutically acceptable salts thereof.

Preferably the DFMO will comprise at least about 70% or 80%, more preferably at least about 90%, most preferably at least about 95% of the L-DFMO. Ideally, the DFMO will be substantially optically pure L-DFMO. "Substantially optically pure" means that the DFMO comprises at least 98% L-DFMO. "Optically pure" L-DFMO means that the DFMO comprises essentially 100% L-DFMO.

Preferred compositions include about 0.1% to about 30%, preferably about 1% to about 20%, more preferably about 5% to about 15%, by weight of the DFMO.

The present invention also provides topical compositions including a dermatologically or cosmetically acceptable vehicle, urea, and difluoromethylornithine in an amount effective to reduce hair growth.

The above compositions generally have an enhanced efficacy relative to similar compositions having vehicles containing no urea. This enhanced efficacy can manifest itself, for example, in earlier onset of hair growth inhibiting activity, greater reduction of hair growth rate, and/or greater number of subjects demonstrating reduced hair growth.

Other features and advantages of the invention will be apparent from the description and from the claims.

### DETAILED DESCRIPTION

A preferred composition includes DFMO in an amount effective to reduce hair growth in a cosmetically and/or dermatologically acceptable vehicle including at least 1% by weight urea. A more preferred composition will also include at least 2% by weight of a polyoxyethylene ether having the chemical formula R(OCH₂ CH₂)_{b}OH, where R is a saturated or unsaturated alkyl group including from 8 to 20 carbon atoms and b is from 2 to 100. The composition may be a solid, semi-solid, cream or liquid. The composition may be, for example, a cosmetic and dermatologic product in the form of an, for example, ointment, lotion, foam, cream, gel, or solution. The composition may also be in the form of a shaving preparation or an aftershave. The vehicle itself can be inert or it can possess cosmetic, physiological and/or pharmaceutical benefits of its own.

Preferred polyoxyethylene ethers include polyoxyethylene (2) stearyl ether (steareth-2) (R=CH₃(CH₂)₁₇, b=2), polyoxyethylene (2) oleyl ether (oleth-2) (R=CH₃(CH₂)₇ CHCH(CH₂)₈, b=2), polyoxyethylene (4) lauryl ether (laureth-4) (R=CH₃(CH₂)₁₁, b=4), polyoxyethylene (23) lauryl ether (laureth-23) (R=CH₃(CH₂)₁₁, b=23), a mixture of polyoxyethylene (20) cetyl ether and polyoxyethylene (20) stearyl ether (ceteareth-20) (R=CH₃(CH₂)₁₅ and CH₃(CH₂)₁₇, b=20), and polyoxyethylene (20) stearyl ether (steareth-20) (R=CH₃(CH₂)₁₇, b=20).

The composition may include one or more other types of hair growth reducing agents, such as those described in U.S. Pat. 5,364,885 or U.S. Pat. 5,652,273.

The concentration of DFMO in the composition may be varied over a wide range up to a saturated solution, preferably from 0.1% to 30% by weight; the reduction of hair growth increases as the amount of DFMO applied increases per unit area of skin. The maximum amount effectively applied is limited only by the rate at which the DFMO penetrates the skin. The effective amounts may range, for example, from 10 to 3000 micrograms or more per square centimeter of skin.

Vehicles can be formulated with liquid or solid emollients, solvents, thickeners, humectants and/or powders. Emollients include, for example, stearyl alcohol, mink oil, cetyl alcohol, oleyl alcohol, isopropyl laurate, polyethylene glycol, olive oil, petroleum jelly, palmitic acid, oleic acid, and myristyl myristate. Solvents include, for example, water, ethyl alcohol, isopropanol, acetone, diethylene glycol, ethylene glycol, dimethyl sulfoxide, and dimethyl formamide.

Optically pure L-DFMO can be prepared by known methods. See, for example, U.S. Pat. 4,309,442, Gao et al., Ann. Pharm. Fr. 52(4):184-203 (1994); Gao et al., Ann. Pharm. Fr. 52(5):248-59 (1994); and Jacques et al., Tetrahedron Letters, 48:4617 (1971).

The following are examples of compositions.

### EXAMPLES 1-4

Examples of DFMO formulations containing urea with or without a polyoxyethylene ether.

| Ingredient | Example - 1 | Example - 2 | Example - 3 | Example - 4 |
|---|---|---|---|---|
| | Percent (wt/wt) | Percent (wt/wt) | Percent (wt/wt) | Percent (wt/wt) |
| Water | q.s. | q.s. | q.s. | q.s. |
| Glyceryl Stearate1¹ | 4.16 | 4.24 | 3.94 | 4.24 |
| PEG-100 Stearate¹ | 4.01 | 4.09 | 3.80 | 4.09 |
| Cetearyl Alcohol² | 2.99 | 3.05 | 2.84 | 3.05 |
| Ceteareth-20² | 2.45 | 2.50 | 2.33 | 2.50 |
| Mineral Oil | 2.18 | 2.22 | 2.06 | 2.22 |
| Stearyl Alcohol | 1.64 | 1.67 | 1.55 | 1.67 |
| Dimethicone | 0.55 | 0.56 | 0.52 | 0.56 |
| Preservative³ | 0.4 - 0.78 | 0.4 - 0.78 | 0.4 - 0.78 | 0.4 - 0.78 |
| Urea | 2-5 | 2-5 | 2-5 | 2-5 |
| Polyoxyethylene ether⁴ | ----- | ----- | 5 | 5 |
| Vehicle total | 100% | 100% | 100% | 100% |
| | | | | |
| DFMO⁵ | 1-15% | 1-15% | 1-15% | 1-15% |

| | | | | |
|---|---|---|---|---|
| 1. Available as a blend, for example Cithrol GMS A/S ES0743 from Croda Chemical Company (UK) 2. Available as a blend, for example Cosmowax EM5483 from Croda Chemical Company (UK) 3. Preservative: combination of phenoxyethanol and methyl -, ethyl -, propyl - and butyl - parabens. The preservative is available as premixed blend or as individual ingredients. The preservative could include all of these components or could contain only phenoxyethanol with one or more of the listed parabens. 4. The polyoxyethylene ether may be selected from: ceteareth-20, ceteth-20, steareth-20, oleth-2, steareth-2, laureth-23, or laureth-4, with ceteareth-20 being more preferred. 5. The active drug component DFMO is added at final levels of 1 to 15% to either the pre-emulsified (cream or lotion) vehicle in Examples 1 - 4, or is dissolved first in the water component and then the remaining ingredients are added to form a stable emulsion. After the DFMO addition, the concentrations of other ingredients in the vehicle are accordingly reduced. Preferably the DFMO is substantially optically pure L-DFMO. | | | | |

### EXAMPLE 5

A composition contains up to 15% by weight DFMO in a vehicle containing water 61.2%, ethanol 14.4%, urea 5.0%, steareth-20 5.0%, propylene glycol 4.5%, dipropylene glycol 4.5%, benzyl alcohol 3.6%, and propylene carbonate 1.8%. In place of steareth-20, there can be substituted oleth-2, steareth-2, ceteth-20, ceteareth-20, laureth-23, or laureth-4.

### EXAMPLE 6

Any one or more of the previous examples in combination with one or more of the penetration enhancers selected from: terpenes (e.g., 3-hydroxy-3,7,11-trimethyl-1,6,10-dodecatriene or nerolidol), propan-2-ol, cis-fatty acids (oleic acid, palmitoleic acid), acetone, laurocapram, dimethyl sulfoxide, 2-pyrrolidone, oleyl alcohol, cholesterol, myristic acid isopropyl ester, and propylene glycol. A penetration enhancer may be added at a concentration of, for example, 0.10% to 20% by weight.

The composition should be topically applied to a selected area of the body from which it is desired to reduce hair growth. For example, the composition can be applied to the face, particularly to the beard area of the face, i.e., the cheek, neck, upper lip, or chin. The composition also may be used as an adjunct to other methods of hair removal including shaving, waxing, mechanical epilation, chemical depilation, electrolysis and laser-assisted hair removal.

The composition can also be applied to the legs, arms, torso or armpits. The composition is particularly suitable for reducing the growth of unwanted hair in women, particularly unwanted facial hair, for example, on the upper lip or chin. The composition should be applied once or twice a day, or even more frequently, to achieve a perceived reduction in hair growth. Perception of reduced hair growth can occur as early as 24 hours or 48 hours (for instance, between normal shaving intervals) following use or can take up, to, for example, three months. Reduction in hair growth is demonstrated when, for example, the rate of hair growth is slowed, the need for removal is reduced, the subject perceives less hair on the treated site, or quantitatively, when the weight of hair removed (i.e., hair mass) is reduced (quantitatively), subjects perceive a reduction, for example, in facial hair, or subjects are less concerned or bothered about their unwanted hair (e.g., facial hair).

### SKIN PENETRATION ASSAY

An in vitro diffusion assay for vehicles was established based on that reported by Franz, Curr. Prol. Dermat. 7:58-68 (1978). Dorsal skin from Golden Syrian hamsters was clipped with electric clippers, trimmed to the appropriate size and placed in a diffusion chamber. The receptor fluid consisted of phosphate buffered saline, an isotonic solution for maintaining cell viability and 0.1 % sodium azide, a preservative, and was placed in the lower chamber of the diffusion apparatus such that the level of the fluid was equal to the skin. After equilibration at 37°C for at least 30 minutes, 10 µl or 20 µl of ¹⁴C-DFMO (0.5 to 1.0 T Ci per diffusion chamber) in a test or control formulation was added to the surface of the skin and gently spread over the entire surface with a glass stirring rod. Penetration of DFMO was assessed by periodically removing an aliquot (400 TL) throughout the course of the experiment, and quantitating using liquid scintillation.

This assay was conducted on the vehicle described in Example 1 (with 2% urea). The vehicle not including urea was used as the control. The urea increased DFMO skin penetration over 2-fold after two hours and about 1.5-fold after 24 hours.

The assay also was conducted on the vehicle described in Example 3 (with 2% urea) where the polyoxyethylene ether was selected from either laureth-4, steareth-20 or ceteareth-20. The vehicle not including the urea or the further quantity of polyoxyethylene ether was used as the control. For these vehicles, DFMO penetration was enhanced about 2-fold or more after six hours.

The assay was also conducted on the vehicle described in Example 5. The vehicle not including the urea or the steareth-20 was used as the control. The DFMO penetration was increased by about 3-fold after two or six hours.

Other embodiments are within the scope of the following claims.

## Claims

1. A non-therapeutic, cosmetic method of reducing human hair growth, comprising
selecting an area of skin from which reduced hair growth is desired, and
applying to the area of skin, in an amount effective to reduce hair growth, a composition including α-difluoromethylornithine and a dermatologically acceptable vehicle comprising urea.

2. The method of claim 1, wherein the vehicle includes from 0.1% to 20% by weight urea.

3. The method of claim 1, wherein the vehicle includes from 1% to 12% by weight urea.

4. The method of claim 1, wherein the vehicle includes from 2% to 10% by weight urea.

5. The method of claim 2, wherein the vehicle includes at least 4% by weight urea.

6. The method of claim 1, wherein the vehicle further comprises a polyoxyethylene ether having the chemical formula R(OCH₂CH₂)_{b} OH, where R is a saturated or unsaturated alkyl group including from 6 to 22 carbon atoms and b is from 2 to 200.

7. The method of claim 6, wherein the vehicle includes from 0.1% to 20% by weight of the polyoxyethylene ether.

8. The method of claim 7, wherein the vehicle includes at least 2% by weight of the polyoxyethylene ether.

9. The method of claim 7, wherein the vehicle includes at least 4% by weight of the polyoxyethylene ether.

10. The method of claim 9, wherein the vehicle includes from 1% to 12% by weight urea.

11. The method of claim 10, wherein the vehicle includes at least 2% by weight urea.

12. The method of claim 10, wherein the vehicle includes at least 4% by weight urea.

13. The method of claim 12, wherein the composition includes from 5% to 20% by weight α-difluoromethylornithine.

14. The method of claim 13, wherein the α-difluoromethylornithine is substantially optically pure L-α-difluoromethylornithine.

15. The method of claim 6, wherein R includes from 10 to 20 carbon atoms.

16. The method of claim 6, wherein b has an average value of from 2 to 50.

17. The method of claim 6, wherein the polyoxyethylene ether is selected from the group consisting of steareth-2, oleth-2, laureth-4, laureth-23, ceteth-20, steareth-20, ceteareth-20, and mixtures of two or more of thesepolyoxyethylene ether.

18. The method of claim 6, wherein the polyoxyethylene ether is ceteareth-20.

19. The method of claim 1, wherein the α-difluoromethylornithine comprises at least about 80% of L-α-difluoromethylornithine.

20. The method of claim 1, wherein the α-difluoromethylornithine comprises at least about 95% of L-α-difluoromethylornithine.

21. The method of claim 1, wherein the area of skin is on the face.

22. A composition for topical application to the skin, comprising αdifluoromethylornithine in an amount effective to reduce hair growth and a dermatologically acceptable vehicle comprising urea.

23. The composition of claim 22, wherein the vehicle includes from 1% to 12% by weight urea.

24. The composition of claim 23, wherein the vehicle includes at least 2% by weight urea.

25. The composition of claim 23, wherein the vehicle includes at least 4% by weight urea.

26. The composition of claim 22, wherein the vehicle further comprises a polyoxyethylene ether having the chemical formula R(OCH₂CH₂)_{b} OH, where R is a saturated or unsaturated alkyl group including from 6 to 22 carbon atoms and b is from 2 to 200.

27. The composition of claim 26, wherein the vehicle includes from 2% to 20% by weight of the polyoxyethylene ether.

28. The composition of claim 27, wherein the vehicle includes at least 4% by weight of the polyoxyethylene ether.

29. The composition of claim 26, wherein the vehicle includes from 2% to 12% by weight urea.

30. The composition of claim 29, wherein the vehicle includes at least 4% by weight of the urea and the composition includes from 5% to 15% by weight substantially optically pure L-α-difluoromethylornithine.

31. The composition of claim 26, wherein R includes from 10 to 20 carbon atoms and b is from 2 to 50.

32. The composition of claim 26, wherein the polyoxyethylene ether is selected from the group consisting of steareth-2, oleth-2, laureth-4, laureth-23, ceteth-20, steareth-20, ceteareth-20, and mixtures of two or more of thesepolyoxyethylene ether.

33. The composition of claim 26, wherein the polyoxyethylene ether is ceteareth-20.

34. The composition of claim 22, wherein the α-difluoromethylornithine ornithine comprises at least about 80% L-α-difluoromethylornithine.

35. The composition of claim 22, wherein the α-difluoromethylornithine comprises at least about 95% L-α-difluoromethylornithine.

36. The method according to any one of claims 1 to 21, wherein said applying of said inhibitor has a cosmetic effect.

37. The composition according to any one of claims 22 to 35, which is a cosmetic composition.

38. The use of urea for the manufacture of a medicament comprising a α-difluoromethylornithine reducing human hair growth.

39. The use according to claim 38, wherein said medicament includes a dermatologically acceptable vehicle.

40. The use according to claim 39, wherein the medicament is as defined in any one of claims 2 to 20.

41. A method of producing a composition for inhibiting mammalian hair growth, which comprises admixing urea with α-difluoromethylornithine, said α-difluoromethylornithine being in an amount effective to reduce hair growth, and with a non-toxic, dermatologically acceptable vehicle or carrier.

42. A method according to claim 41, wherein a cosmetic composition is produced.

43. A method according to claim 41, wherein said composition is as defined in any one of claims 23 to 35.

## Patentansprüche

1. Nichttherapeutisches, kosmetisches Verfahren zum Reduzieren von menschlichem Haarwuchs, umfassend
Auswählen eines Hautbereichs, bei dem reduzierter Haarwuchs gewünscht wird, und
Auftragen einer Zusammensetzung, die α-Difluormethylornithin und einen hautverträglichen Träger, der Harnstoff umfasst, enthält, auf den Hautbereich in einer zum Reduzieren des Haarwuchses wirksamen Menge.

2. Verfahren nach Anspruch 1, wobei der Träger 0,1 Gew.-% bis 20 Gew.-% Harnstoff enthält.

3. Verfahren nach Anspruch 1, wobei der Träger 1 Gew.-% bis 12 Gew.-% Harnstoff enthält.

4. Verfahren nach Anspruch 1, wobei der Träger 2 Gew.-% bis 10 Gew.-% Harnstoff enthält.

5. Verfahren nach Anspruch 2, wobei der Träger zu mindestens 4 Gew.-% Harnstoff enthält.

6. Verfahren nach Anspruch 1, wobei der Träger ferner einen Polyoxyethylenether mit der chemischen Formel R(OCH₂CH₂)_{b}OH umfasst, worin R eine gesättigte oder ungesättigte Alkylgruppe mit 6 bis 22 Kohlenstoffatomen ist und b 2 bis 200 ist.

7. Verfahren nach Anspruch 6, wobei der Träger 0,1 Gew.-% bis 20 Gew.-% des Polyoxyethylenethers enthält.

8. Verfahren nach Anspruch 7, wobei der Träger zu mindestens 2 Gew.-% den Polyoxyethylenether enthält.

9. Verfahren nach Anspruch 7, wobei der Träger zu mindestens 4 Gew.-% den Polyoxyethylenether enthält.

10. Verfahren nach Anspruch 9, wobei der Träger 1 Gew.-% bis 12 Gew.-% Harnstoff enthält.

11. Verfahren nach Anspruch 10, wobei der Träger zu mindestens 2 Gew.-% Harnstoff enthält.

12. Verfahren nach Anspruch 10, wobei der Träger zu mindestens 4 Gew.-% Harnstoff enthält.

13. Verfahren nach Anspruch 12, wobei die Zusammensetzung 5 Gew.-% bis 20 Gew.-% α-Difluormethylornithin enthält.

14. Verfahren nach Anspruch 13, wobei das α-Difluormethylornithin im Wesentlichen optisch reines L-α-Difluormethylornithin ist.

15. Verfahren nach Anspruch 6, wobei R 10 bis 20 Kohlenstoffatome enthält.

16. Verfahren nach Anspruch 6, wobei b einen durchschnittlichen Wert von 2 bis 50 aufweist.

17. Verfahren nach Anspruch 6, wobei der Polyoxyethylenether ausgewählt ist aus der Gruppe, bestehend aus Steareth-2, Oleth-2, Laureth-4, Laureth-23, Ceteth-20, Steareth-20, Ceteareth-20 und Mischungen von zwei oder mehr dieser Polyoxyethylenether.

18. Verfahren nach Anspruch 6, wobei der Polyoxyethylenether Ceteareth-20 ist.

19. Verfahren nach Anspruch 1, wobei das α-Difluormethylornithin zu mindestens ungefähr 80 % L-α-Difluormethylornithin umfasst.

20. Verfahren nach Anspruch 1, wobei das α-Difluormethylornithin zu mindestens ungefähr 95 % L-α-Difluormethylornithin umfasst.

21. Verfahren nach Anspruch 1, wobei der Hautbereich auf dem Gesicht ist.

22. Zusammensetzung zur äußerlichen Anwendung auf der Haut, umfassend α-Difluormethylornithin in einer zum Reduzieren des Haarwuchses wirksamen Menge und einen hautverträglichen Träger, der Harnstoff umfasst.

23. Zusammensetzung nach Anspruch 22, wobei der Träger 1 Gew.-% bis 12 Gew.-% Harnstoff enthält.

24. Zusammensetzung nach Anspruch 23, wobei der Träger zu mindestens 2 Gew.-% Harnstoff enthält.

25. Zusammensetzung nach Anspruch 23, wobei der Träger zu mindestens 4 Gew.-% Harnstoff enthält.

26. Zusammensetzung nach Anspruch 22, wobei der Träger ferner einen Polyoxyethylenether mit der chemischen Formel R(OCH₂CH₂)_{b}OH umfasst, worin R eine gesättigte oder ungesättigte Alkylgruppe mit 6 bis 22 Kohlenstoffatomen ist und b 2 bis 200 ist.

27. Zusammensetzung nach Anspruch 26, wobei der Träger 2 Gew.-% bis 20 Gew.-% des Polyoxyethylenethers enthält.

28. Zusammensetzung nach Anspruch 27, wobei der Träger zu mindestens 4 Gew.-% den Polyoxyethylenether enthält.

29. Zusammensetzung nach Anspruch 26, wobei der Träger von 2 Gew.-% bis 12 Gew.-% Harnstoff enthält.

30. Zusammensetzung nach Anspruch 29, wobei der Träger zu mindestens 4 Gew.-% den Harnstoff enthält und die Zusammensetzung 5 Gew.-% bis 15 Gew.-% im Wesentlichen optisch reines L-α-Difluormethylornithin enthält.

31. Zusammensetzung nach Anspruch 26, wobei R 10 bis 20 Kohlenstoffatome enthält und b 2 bis 50 ist.

32. Zusammensetzung nach Anspruch 26, wobei der Polyoxyethylenether ausgewählt ist aus der Gruppe, bestehend aus Steareth-2, Oleth-2, Laureth-4, Laureth-23, Ceteth-20, Steareth-20, Ceteareth-20 und Mischungen von zwei oder mehr dieser Polyoxyethylenether.

33. Zusammensetzung nach Anspruch 26, wobei der Polyoxyethylenether Ceteareth-20 ist.

34. Zusammensetzung nach Anspruch 22, wobei das α-Difluormethylornithin zu mindestens ungefähr 80 % L-α-Difluormethylornithin umfasst.

35. Zusammensetzung nach Anspruch 22, wobei das α-Difluormethylornithin zu mindestens ungefähr 95 % L-α-Difluormethylornithin umfasst.

36. Verfahren nach einem der Ansprüche 1 bis 21, worin das Auftragen des Inhibitors eine kosmetische Wirkung hat.

37. Zusammensetzung nach einem der Ansprüche 22 bis 35, die eine Kosmetikzusammensetzung ist.

38. Verwendung von Harnstoff zur Herstellung eines Medikaments, umfassend ein α-Difluormethylornithin, das menschlichen Haarwuchs reduziert.

39. Verwendung nach Anspruch 38, wobei das Medikament einen hautverträglichen Träger enthält.

40. Verwendung nach Anspruch 39, wobei das Medikament wie in einem der Ansprüche 2 bis 20 definiert ist.

41. Verfahren zum Herstellen einer Zusammensetzung zum Hemmen von Säugetier-Haarwuchs, das das Mischen von Harnstoff mit α-Difluormethylornithin, wobei das α-Difluormethylornithin in einer zum Reduzieren des Haarwuchses wirksamen Menge vorliegt, und mit einem nichttoxischen, hautverträglichen Medium oder Träger umfasst.

42. Verfahren nach Anspruch 41, wobei eine Kosmetikzusammensetzung erzeugt wird.

43. Verfahren nach Anspruch 41, wobei die Zusammensetzung wie in einem der Ansprüche 23 bis 35 definiert ist.

## Revendications

1. Procédé cosmétique non thérapeutique de réduction de la pousse des poils humains, comprenant
le choix d'une zone de peau de laquelle une pousse des poils réduite est souhaitée, et
l'application sur la zone de peau, en une quantité efficace pour réduire la pousse des poils, d'une composition incluant de l'α-difluorométhylornithine et un véhicule acceptable du point de vue dermatologique comprenant de l'urée.

2. Procédé selon la revendication 1, dans lequel le véhicule inclut de 0,1 % à 20 % en poids d'urée.

3. Procédé selon la revendication 1, dans lequel le véhicule inclut de 1 % à 12 % en poids d'urée.

4. Procédé selon la revendication 1, dans lequel le véhicule inclut de 2 % à 10 % en poids d'urée.

5. Procédé selon la revendication 2, dans lequel le véhicule inclut au moins 4 % en poids d'urée.

6. Procédé selon la revendication 1, dans lequel le véhicule comprend, en outre, un polyoxyéthylène éther ayant la formule chimique R(OCH₂CH₂)_{b} OH, où R est un groupe alkyle saturé ou insaturé incluant de 6 à 22 atomes de carbone et b va de 2 à 200.

7. Procédé selon la revendication 6, dans lequel le véhicule inclut de 0,1 % à 20 % en poids du polyoxyéthylène éther.

8. Procédé selon la revendication 7, dans lequel le véhicule inclut au moins 2 % en poids du polyoxyéthylène éther.

9. Procédé selon la revendication 7, dans lequel le véhicule inclut au moins 4 % en poids du polyoxyéthylène éther.

10. Procédé selon la revendication 9, dans lequel le véhicule inclut de 1 % à 12 % en poids d'urée.

11. Procédé selon la revendication 10, dans lequel le véhicule inclut au moins 2 % en poids d'urée.

12. Procédé selon la revendication 10, dans lequel le véhicule inclut au moins 4 % en poids d'urée.

13. Procédé selon la revendication 12, dans lequel la composition inclut de 5 % à 20 % en poids d'α-difluorométhylornithine.

14. Procédé selon la revendication 13, dans lequel l'α-difluorométhylornithine est une L-α-difluorométhylomithine essentiellement optiquement pure.

15. Procédé selon la revendication 6, dans lequel R inclut de 10 à 20 atomes de carbone.

16. Procédé selon la revendication 6, dans lequel b a une valeur moyenne allant de 2 à 50.

17. Procédé selon la revendication 6, dans lequel le polyoxyéthylène éther est choisi dans le groupe constitué de stéareth-2, oléth-2, lauréth-4, lauréth-23, céteth-20, stéareth-20, cétéareth-20, et des mélanges de deux ou plusieurs de ces polyoxyéthylène éthers.

18. Procédé selon la revendication 6, dans lequel le polyoxyéthylène éther est cétéareth-20.

19. Procédé selon la revendication 1, dans lequel l'α-difluorométhylornithine comprend au moins environ 80 % de L-α-difluorométhylornithine.

20. Procédé selon la revendication 1, dans lequel l'α-difluorométhylornithine comprend au moins environ 95 % de L-α-difluorométhylornithine.

21. Procédé selon la revendication 1, dans lequel la zone de peau est sur le visage.

22. Composition pour application locale sur la peau, comprenant de l'α-difluorométhylornithine en une quantité efficace pour réduire la pousse des poils et un véhicule acceptable du point de vue dermatologique comprenant de l'urée.

23. Composition selon la revendication 22, dans laquelle le véhicule inclut de 1 % à 12 % en poids d'urée.

24. Composition selon la revendication 23, dans laquelle le véhicule inclut au moins 2 % en poids d'urée.

25. Composition selon la revendication 23, dans laquelle le véhicule inclut au moins 4 % en poids d'urée.

26. Composition selon la revendication 22, dans lequel le véhicule comprend en outre un polyoxyéthylène éther ayant la formule chimique R(OCH₂CH₂)_{b} OH, où R est un groupe alkyle saturé ou insaturé incluant de 6 à 22 atomes de carbone et b va de 2 à 200.

27. Composition selon la revendication 26, dans laquelle le véhicule inclut de 2 % à 20 % en poids du polyoxyéthylène éther.

28. Composition selon la revendication 27, dans laquelle le véhicule inclut au moins 4 % en poids du polyoxyéthylène éther.

29. Composition selon la revendication 26, dans laquelle le véhicule inclut de 2 % à 12 % en poids d'urée.

30. Composition selon la revendication 29, dans laquelle le véhicule inclut au moins 4 % en poids de l'urée et la composition inclut de 5 % à 15 % en poids de L-α-difluorométhylornithine essentiellement optiquement pure.

31. Composition selon la revendication 26, dans laquelle R inclut de 10 à 20 atomes de carbone et b va de 2 à 50.

32. Composition selon la revendication 26, dans laquelle le polyoxyéthylène éther est choisi dans le groupe constitué de stéareth-2, oléth-2, lauréth-4, lauréth-23, céteth-20, stéareth-20, cétéareth-20, et des mélanges de deux ou plus de ces polyoxyéthylène éthers.

33. Composition selon la revendication 26, dans laquelle le polyoxyéthylène éther est cétéareth-20.

34. Composition selon la revendication 22, dans laquelle l'α-difluorométhylornithine ornithine comprend au moins environ 80 % de L-α-difluorométhylornithine.

35. Composition selon la revendication 22, dans laquelle l'α-difluorométhylornithine comprend au moins environ 95 % de L-α-difluorométhylornithine.

36. Procédé selon l'une quelconque des revendications 1 à 21, dans lequel ladite application dudit inhibiteur a un effet cosmétique.

37. Composition selon l'une quelconque des revendications 22 à 35, qui est une composition cosmétique.

38. Utilisation d'urée pour la fabrication d'un médicament comprenant une α-difluorométhylornithine réduisant la pousse des poils humains.

39. Utilisation selon la revendication 38, dans laquelle ledit médicament inclut un véhicule acceptable du point de vue dermatologique.

40. Utilisation selon la revendication 39, dans laquelle le médicament est tel que défini dans l'une quelconque des revendications 2 à 20.

41. Procédé de production d'une composition pour inhiber la pousse des poils de mammifères, qui comprend un mélange d'urée avec de l'α-difluorométhylornithine, ladite α-difluorométhylornithine étant en une quantité efficace pour réduire la pousse des poils, et avec un véhicule ou support non-toxique, acceptable du point de vue dermatologique.

42. Procédé selon la revendication 41, dans lequel une composition cosmétique est produite.

43. Procédé selon la revendication 41, dans lequel ladite composition est telle que définie dans l'une quelconque des revendications 23 à 35.
